# EUROPEAN PATENT SPECIFICATION

(11) **EP 1 572 616 B1**
(45) Date of publication and mention of the grant of the patent: **11.04.2007**
(21) Application number: 03814614.8
(22) Date of filing: 23.10.2003
(51) Int. Cl.: C07C 51/58, C07C 59/315

(54) **SELECTIVE REACTION OF HEXAFLUOROPROPYLENE OXIDE WITH PERFLUOROACYL FLUORIDES**
SELEKTIVE REAKTION VON HEXAFLUORPROPYLENOXID MIT PERFLUORACYLFLUORIDEN
REACTION SELECTIVE D'OXYDE D'HEXAFLUOROPROPYLENE AVEC DES FLUORURES DE PERFLUOROACYLE

(30) Priority: 17.12.2002 US 322254
(43) Date of publication of application: 14.09.2005
(73) Proprietor: 3M Innovative Properties Company, St. Paul, MN 55133-3427 (US)
(72) Inventor: GUERRA, Miguel, A., Saint Paul, MN 55133-3427 (US)
(74) Representative: Vossius & Partner
(86) International application number: PCT/US2003/033958
(87) International publication number: WO 2004/060849

(56) References cited:
- US-A- 3 250 808
- US-A- 4 749 526

## Description

This invention relates to a process for reacting hexafluoropropylene oxide (HFPO) with a perfluoroacyl fluorides according to the formula X-R_{f}-COF to selectively produce the monoaddition product according to the formula X-R_{f}-CF₂-O-CF(CF₃)COF with high utilization of reactants.

Reactions of hexafluoropropylene oxide (HFPO) with perfluoroacyl fluorides that result in a mixture of reaction products, differing in the number of incorporated HFPO units, are known. In the practice of these reactions, careful fractionation of the product mixture may be required to remove undesirable byproducts, such as products incorporating more than one HFPO unit.

US 4,749,526 discloses preparations for fluoroaliphatic ether-containing carbonyl fluoride compounds by reacting a fluorinated carbonyl compound with hexafluoropropylene oxide in the presence of at least one catalyst selected from potassium iodide, potassium bromide, cesium iodide, cesium bromide, rubidium iodide and rubidium bromide. US-A- 3250 808 discloses fluorocarbon ethers derived from hexafluoropropylene epoxyide.

Briefly, the present invention provides a continuous or repeated-batch process for preparation of a compound according to formula (I): X-R_{f}-CF₂-O-CF(CF₃)COF, wherein X- is F-, FOC- or FSO₂- and wherein -R_{f}- is a linear, branched or cyclic fluoroalkene group containing 1-20 carbon atoms which is highly fluorinated and which may incorporate ether and tertiary amine groups, comprising the steps of: a) providing a mixture of X-R_{f}-COF (II), wherein X- and -R_{f}- are as defined for formula (I), a fluoride salt, and a polar solvent; b) adding hexafluoropropylene oxide (HFPO) in an amount such that X-R_{f}-COF remains in molar excess of HFPO by at least 10% and reacting X-R_{f}-COF with HFPO; c) separating unreacted X-R_{f}-COF from a mixture of addition products of hexafluoropropylene oxide (HFPO) and X-R_{f}-COF; and d) repeating step a) using unreacted X-R_{f}-COF separated in step c).

In another aspect, the present invention provides a method of reacting hexafluoropropylene oxide (HFPO) with perfluoroacyl fluorides according to the formula X-R_{f}-COF (II), wherein X and -R_{f}- are as described above, to form a mixture of addition products comprising the monoaddition product according to the formula X-R_{f}-CF₂-O-CF(CF₃)COF (I), wherein the molar amount of the monoaddition product is 90% or greater of the combined molar amount of the monoaddition product and a biaddition product according to the formula X-R_{f}-CF₂-O-CF(CF₃)CF₂-O-CF(CF₃)COF (III) in the mixture of addition products. More typically, the molar amount of the monoaddition product is 95% or greater of the combined molar amount of the monoaddition and biaddition products in the mixture of addition products.

What has not been described in the art, and is provided by the present invention, is an industrially useful method to react hexafluoropropylene oxide (HFPO) with perfluoroacyl fluorides which provides high selectivity for the monoaddition product, and, when excess perfluoroacyl fluoride is recycled, provides utilization of both HFPO and the perfluoroacyl fluoride reactant that approaches the level of selectivity, i.e., in excess of 90% and more typically in excess of 95%.

In this application, "highly fluorinated" means containing fluorine in an amount of 40 wt% or more, typically 50 wt% or more and more typically 60 wt% or more.

It is an advantage of the present invention to provide an industrially advantageous method of reacting hexafluoropropylene oxide (HFPO) with perfluoroacyl fluorides that provides utilization of both HFPO and the perfluoroacyl fluoride reactant that approaches 90% or higher.

The present invention provides a continuous or repeated-batch process for preparation of a compound according to formula (I): X-R_{f}-CF₂-O-CF(CF₃)COF, wherein X- is F-, FOC- or FSO₂ - and wherein -R_{f}- is a linear, branched or cyclic fluoroalkene group containing 1-20 carbon atoms which is highly fluorinated and which may incorporate ether and tertiary amine groups, comprising the steps of: a) providing a mixture of X-R_{f}-COF (II), wherein X- and -R_{f}- are as defined for formula (I), a fluoride salt, and a polar solvent; b) adding hexafluoropropylene oxide (HFPO) in an amount such that X-R_{f}-COF remains in molar excess of HFPO by at least 10% and reacting X-R_{f}-COF with HFPO; c) separating unreacted X-R_{f}-COF from a mixture of addition products of hexafluoropropylene oxide (HFPO) and X-R_{f}-COF; and d) repeating step a) using unreacted X-R_{f}-COF separated in step c). In the reaction according to the present invention, the mixture of addition products comprises the monoaddition product according to the formula X-R_{f}-CF₂-O-CF(CF₃)COF (I), resulting from 1:1 combination of HFPO and X-R_{f}-COF, a biaddition product according to the formula X-R_{f}-CF₂-O-CF(CF₃)CF₂-O-CF(CF₃)COF (III), resulting from 2:1 combination of HFPO and X-R_{f}-COF, and potentially products resulting from 3:1,4:1 and higher degrees of addition. Typically, the reaction according to the present invention is selective for the 1:1 product. Typically the molar amount of the monoaddition product is 90% or greater of the combined molar amount of the monoaddition (1:1) product and the biaddition (2:1) product, and more typically 95% or greater.

The perfluoroacyl fluoride reactant is a compound according to the formula:

X-R_{f}-COF (II)

wherein X- is F-, FOC- or FSO₂ - and wherein -R_{f}- is a linear, branched or cyclic fluoroalkene group, typically a linear group, containing 1-20 carbon atoms, typically containing 1-10 carbon atoms, and more typically containing 2-4 carbon atoms, which is highly fluorinated, typically perfluorinated, and which may incorporate ether and tertiary amine groups, but typically incorporates no tertiary amine groups, more typically incorporates no ether or tertiary amine groups.

Any suitable reaction vessel may be used, as appropriate to a continuous or batchwise process. Typically, the process is a continuous or a repeating batch process, allowing for the recovery and reuse of perfluoroacyl fluoride reactant in subsequent repetitions of the reaction. The perfluoroacyl fluoride reactant is mixed with a fluoride salt in a polar solvent to form a pre-reaction mixture. Any suitable fluoride salt may be used, including salts of mono- or polyvalent cations and salts of polyatomic cations or, more typically, monoatomic cations, most typically KF. Typically the salt is provided in an amount of 0.1-10% by weight relative to the amount of perfluoroacyl fluoride reactant, more typically 1-5%, and most typically 2-4%. Any suitable polar solvent may be used. Typically the solvent is provided in an amount of 10-200% by weight relative to the amount of perfluoroacyl fluoride reactant, more typically 20-40%, and most typically 20-30%.

Hexafluoropropylene oxide (HFPO) is added to form a reaction mixture. HFPO is added in an amount such that X-R_{f}-COF remains in molar excess of HFPO by at least about 10%, more typically by at least about 20%, and most typically by at least about 30%. Typically, X-R_{f}-COF is in molar excess of HFPO by no more than 50% after addition of all HFPO.

The reaction mixture may be maintained at any suitable temperature and pressure. Typically, the reaction mixture is maintained at a temperature between -25 °C and 40 °C, more typically between -25 °C and 25 °C, and most typically between -20 °C and 0 °C. Typically, the reaction mixture is maintained at a pressure between vacuum and 300kPa, more typically between 20 and 110 kPa. HFPO may be added at any rate, provided that the temperature does not rise to a level that produces significant unwanted HFPO oligimerization. HFPO may be added very quickly if appropriate cooling apparatus are used.

After completion of the reaction, the unreacted X-R_{f}-COF is typically separated from the mixture of addition products by any suitable means, including solvent separation and distillation. Typically the unreacted X-R_{f}-COF thus recovered is used in a subsequent reaction.

The addition product mixture comprises the monoaddition product according to the formula X-R_{f}-CF₂-O-CF(CF₃)COF (I), resulting from 1:1 combination of HFPO and X-R_{f}-COF, a biaddition product according to the formula X-R_{f}-CF₂-O-CF(CF₃)CF₂-O-CF(CF₃)COF (III), resulting from 2:1 combination of HFPO and X-R_{f}-COF, and potentially, but not typically, products resulting from 3:1, 4:1 and higher degrees of addition. The product mixture may also include low levels, typically <1%, of HFPO dimer, trimer and higher oligomers. The reaction according to the present invention is selective for the 1:1 product, such that the molar amount of the monoaddition product is typically 90% or greater of the combined molar amount of the monoaddition (1:1) product and the biaddition (2:1) product, and more typically 95% or greater. HPFO, a valuable reactant, is productively consumed in an amount approaching but less than the reaction selectivity, since polyaddition of HPFO consumes a disproportionate amount of HPFO. Furthermore, when excess perfluoroacyl fluoride is recycled, utilization of perfluoroacyl fluoride reactant also approaches the reaction selectivity. The potential for reusing unreacted perfluoroacyl fluoride reactant, the high utilization of both reactants, approaching 90% or higher, and the elimination or reduction of cleanup of the monoaddition product all render the process according to the present invention highly useful in industrial applications.

This invention is useful in the industrial synthesis of HFPO-perfluoroacyl fluoride adducts.

Objects and advantages of this invention are further illustrated by the following examples, but the particular materials and amounts thereof recited in these examples, as well as other conditions and details, should not be construed to unduly limit this invention.

### Examples

Unless otherwise noted, all reagents were obtained or are available from Aldrich Chemical Co., Milwaukee, WI, or may be synthesized by known methods.

### Example 1

113g KF and 1960g of diglyme were charged in a 2-gallon (7571 ml) stainless steel reactor and stirred and cooled to -17 °C. 3240 g (11.7 moles) of 4-(fluorosulfonyl)hexafluorobutyryl fluoride, FSO₂CF₂CF₂CF₂COF (M.W. 280.1), prepared by electrochemical fluorination of 1,4-Butane sultone as described in U.S. Pat. No. 2,732,398, was vacuum charged into the reactor and stirred for 30 minutes. (3240 g weight was net of 5807g of 55.8% purity material.) 1485g (8.95 moles) hexafluoropropylene oxide (HFPO) (M.W. 166.0), was added over one hour with the reaction temperature rising to -4oC and pressure up to 28kPa. The molar ratio was 11.7/8.95 = 1.29. The reaction mixture was stirred for 30 minutes and allowed to warm to room temperature. 7350g of a bottom fluorochemical phase was recovered, containing 10% by weight unreacted acid fluoride, 45% by weight of the 1:1 addition product, perfluoro-4-(fluorosulfonyl)butoxypropionyl fluoride, FSO₂-CF₂CF₂CF₂CF₂-O-CF(CF₃)COF and 1.8% by weight of the 2:1 addition byproduct, FSO₂-CF₂CF₂CF₂CF₂-O-CF(CF₃)CF₂-O-CF(CF₃)COF. This result demonstrates an 83% yield based on HFPO (moles 1:1 addition product/moles HFPO reactant) and a desired selectivity for the 1:1 addition product of 96% (moles 1:1 addition product/moles 1:1 and 2:1 product).

### Example 1C (Comparative)

9g KF and 200ml of diglyme were charged in a 600 ml stainless steel reactor and stirred and cooled to 0°C. 180 g (0.64 moles) of 4-(fluorosulfonyl)hexafluorobutyryl fluoride, FSO₂CF₂CF₂CF₂COF (M.W. 280.1) was vacuum charged into the reactor and stirred for 30 minutes. (180 g weight was net of 400g of 45% purity material.) 107g (0.64 moles) hexafluoropropylene oxide (HFPO) (M.W. 166.0), was added over two and one half hours. The molar ratio was 0.64/0.64 = 1.00. The reaction mixture was stirred for 30 minutes and the bottom fluorochemical phase was distilled to give a 210g fraction with a boiling point greater than 110°C containing 75% by weight of the 1:1 1 addition product, 23% by weight of the 2:1 addition byproduct and 2% by weight of the 3:1 addition byproduct. This result demonstrates an 55% yield based on HFPO but a selectivity for the 1:1 1 addition product of 77%, comparing 1:1 1 and 2:1 addition products.

### Example 2

73g KF and 3386g of diglyme were charged in a 2-gallon (7571 ml) stainless steel reactor and cooled to -5 °C. 1693 g (7.30 moles) of perfluoromethoxypropionyl fluoride, CF₃-O-CF₂CF₂COF (M.W. 232.0), prepared as described in U.S. Pat. No. 6,482,979, was vacuum charged into the reactor and stirred for 30 minutes. 848g (5.11 moles) hexafluoropropylene oxide (HFPO) (M.W. 166.0), was added over fifteen minutes and allowed to react for an additional 15 minutes. The molar ratio was 7.30/5.11 = 1.43. The lower fluorochemical phase was distilled to give 1099g of precut containing 26.2% starting acid fluoride and 16% of the desired 1:1 addition product, perfluoromethoxypropoxylpropionyl fluoride, CF₃-O-CF₂CF₂CF₂-O-CF(CF₃)COF. The product cut of 1533g contained 90% by weight of the desired 1:1 addition product, and the final cut of 141g was 66% 2:1 byproduct. This result demonstrates an 77% yield based on HFPO and a desired selectivity for the 1:1 addition product of 94%.

### Example 2C (Comparative)

4.3g KF and 188g of diglyme were charged in a 600 ml stainless steel reactor along with 112 g (0.48 moles) of perfluoromethoxypropionyl fluoride, CF₃-O-CF₂CF₂COF (M.W. 232.0) and the mixture was cooled to 0 °C. 80.2g (0.48 moles) hexafluoropropylene oxide (HFPO) (M. W. 166.0), was added over one hour at 10 °C. The molar ratio was 0.48/0.48 = 1.00. After phase split, 182g of fluorochemical was collected containing 78% of the desired 1:1 addition product and 23% of the 2:1 byproduct for an 74% yield based on HFPO and a selectivity of only 78%.

### Example 3

22g KF and 300g of diglyme were charged in a 2 liter 3-neck round bottom flask along with 1000g (3.40 moles) of perfluoroadipoyl fluoride, FOC-CF₂CF₂CF₂CF₂-COF (M.W. 294.0), prepared by electrochemical fluorination of dimethyl adipate as described in U.S. Pat. No. 6,482,979, and the mixture was stirred for 30 minutes and cooled to -17 °C. 480g (2.89 moles) hexafluoropropylene oxide (HFPO) (M.W. 166.0), was added over one hour at 5 °C and the reaction mixture was stirred for an additional 30 minutes. The molar ratio was 3.40/2.89 = 1.18. 1490g of a bottom fluorochemical phase was recovered, containing 69% by weight of the desired 1:1 addition product, perfluoro-6-(fluoroacyl)hexoxypropionyl fluoride, FOC-CF₂CF₂CF₂CF₂CF₂-O-CF(CF₃)COF and 8.8% by weight of the 2:1 addition byproduct, FOC-CF₂CF₂CF₂CF₂CF₂-O-CF(CF₃)CF₂-O-CF(CF₃)COF. This result demonstrates an 78% yield based on HFPO and a desired selectivity for the 1:1 addition product of 89%.

### Example 3C (Comparative)

33g KF and 2140g of diglyme were charged in a 5 liter 3-neck round bottom flask along with 1043g (3.55 moles) ofperfluoroadipoyl fluoride, FOC-CF₂CF₂CF₂CF₂-COF (M.W. 294.0) and the mixture was stirred for 30 minutes and cooled to 0 °C. (1043 g weight was net of 1257g of 83% purity material.) 587g (3.54 moles) hexafluoropropylene oxide (HFPO) (M.W. 166.0), was added over three hour at room temperature. The molar ratio was 3.55/3.54 = 1.00. The mixture was distilled to give 1470g of a mixture comprising 74% by weight of the desired 1:1 addition product and 26% by weight of the 2:1 addition byproduct. This result demonstrates an 67% yield based on HFPO but a desired selectivity for the 1:1 addition product of only 74%.

The reaction conditions for Examples 1, 1C, 2, 2C, 3, and 3C are summarized in Table I and the results are summarized in Table II. "NM" indicates "not measured."

**Table I**

| Ex. | reactant | reactant m.w. | reactant wt (g) | reactant moles | HFPO m.w. | HFPO wt (g) | HFPO moles | molar ratio |
|---|---|---|---|---|---|---|---|---|
| 1 | FSO2-C3F6-OCF | 280.1 | 3,240 | 11.57 | 166.0 | 1485 | 8.95 | 1.29 |
| 1C | FSO2-C3F6-OCF | 280.1 | 180 | 0.64 | 166.0 | 107 | 0.64 | 1.00 |
| 2 | CF3O-C2F4-COF | 232.0 | 1,693 | 7.30 | 166.0 | 848 | 5.11 | 1.43 |
| 2C | CF3O-C2F4-COF | 232.0 | 112 | 0.48 | 166.0 | 80.2 | 0.48 | 1.00 |
| 3 | FOC-C4F8-COF | 294.0 | 1,000 | 3.40 | 166.0 | 480 | 2.89 | 1.18 |
| 3C | FOC-C4F8-COF | 294.0 | 1,043 | 3.55 | 166.0 | 587 | 3.54 | 1.00 |

**Table II**

| Ex. | 1:1 product | 1:1 product m.w. | 1:1 product wt (g) | 1:1 product moles | Molar Yield | 2:1 product | 2:1 product m.w. | 2:1 product wt (g) | 2:1 product moles | Molar Selectivity | reactant collected (g) | reactant collected (moles) |
|---|---|---|---|---|---|---|---|---|---|---|---|---|
| 1 | FSO2-C4F8-O-CF(CF3)COF | 446.1 | 3,308 | 7.42 | 83% | FSO2-C4F8-O-CF(CF3)CF2-O-CF(CF3)COF | 612.1 | 132 | 0.22 | 96% | 735 | 2.62 |
| 1C | FSO2-C4F8-O-CF(CF3)COF | 446.1 | 239 | 0.54 | 55% | FSO2-C4F8-O-CF(CF3)CF2-O-CF(CF3)COF | 612.1 | 73 | 0.12 | 77% | NM | NM |
| 2 | CF3O-C3F6-O-CF(CF3)COF | 398.1 | 1,556 | 3.91 | 77% | CF3O-C3F6-O-CF(CF3)CF2-O-CF(CF3)COF | 564.1 | 93 | 0.16 | 94% | 288 | 1.24 |
| 2C | CF30-C3F6-O-CF(CF3)COF | 398.1 | 141 | 0.35 | 74% | CF30-C3F6-O-CF(CF3)CF2-O-CF(CF3)COF | 564.1 | 41 | 0.07 | 78% | NM | NM |
| 3 | FOC-C5F10-O-CF(CF3)COF | 460.1 | 1,033 | 2.25 | 78% | FOC-C5F10-O-CF(CF3)CF2-O-CF(CF3)COF | 626.1 | 131 | 0.21 | 89% | NM | NM |
| 3C | FOC-C5F10-O-CF(CF3)COF | 460.1 | 1,088 | 2.36 | 67% | FOC-C5F10-O-CF(CF3)CF2-O-CF(CF3)COF | 626.1 | 382 | 0.61 | 74% | NM | NM |

It can be readily seen that the process according to the present invention provides greatly improved selectivity for the 1:1 addition (monoaddition) product, often at greater yield. Furthermore, valuable unreacted perfluoroacyl fluoride reactant can be recovered for reuse, which renders the process according to the present invention highly useful in industrial applications such as continuous or repeated batch processes. When excess perfluoroacyl fluoride is recycled, utilization of perfluoroacyl fluoride reactant approaches the degree of reaction selectivity. Since HFPO, also a valuable reactant, is productively consumed in an amount slightly less than the reaction selectivity, utilization of both HFPO and the perfluoroacyl fluoride reactant approaches the level of selectivity, i.e., in excess of 90% and more typically in excess of 95%.

## Claims

1. A continuous or repeated-batch process for preparation of a compound according to formula (I):
X-R_{f}-CF₂-O-CF(CF₃)COF (I)
wherein X- is F-, FOC- or FSO₂ - and wherein -R_{f}- is a linear, branched or cyclic fluoroalkene group containing 1-20 carbon atoms which is highly fluorinated and which may incorporate ether and tertiary amine groups, comprising the steps of:
a) providing a mixture of:
X-R_{f}-COF (II)
wherein X- and -R_{f}- are as defined for formula (I), a fluoride salt, and a polar solvent;
b) adding hexafluoropropylene oxide (HFPO) in an amount such that X-R_{f}-COF remains in molar excess of HFPO by at least 10% and reacting X-R_{f}-COF with HFPO;
c) separating unreacted X-R_{f}-COF from a mixture of addition products of hexafluoropropylene oxide (HFPO) and X-R_{f}-COF;
d) repeating step a) using unreacted X-R_{f}-COF separated in step c).

2. The method according to claim 1 wherein said mixture of addition products comprise a monoaddition product according to formula (1):
X-R_{f}-CF₂-O-CF(CF₃)COF (I)
and a biaddition product according to formula (III):
X-R_{f}-CF₂-O-CF(CF₃)CF₂-O-CF(CF₃)COF (III)
wherein the molar amount of monoaddition product in said mixture of addition products is 90% or greater of the combined molar amount of said monoaddition product and said biaddition product.

3. The method according to claim 2 wherein molar yield of said monoaddition product relative to moles of HFPO added in step b) is 75% or greater.

4. The method according to any of claims 1-3 wherein step b) is carried out without addition of any catalyst other than said fluoride salt.

5. A method of reacting hexafluoropropylene oxide (HFPO) with a perfluoroacyl fluoride according to the formula:
X-R_{f}-COF (II)
wherein X is F-, FOC- or FSO₂ - and wherein -R_{f}- is a linear, branched or cyclic fluoroalkene group containing 1-20 carbon atoms which is highly fluorinated and which may incorporate ether and tertiary amine groups,
to form a mixture of addition products comprising the monoaddition product according to the formula:
X-R_{f}-CF₂-O-CF(CF₃)COF (I)
wherein the molar amount of said monoaddition product is 90% or greater of the combined molar amount of said monoaddition product and a biaddition product according to the formula:
X-R_{f}-CF₂-O-CF(CF₃)CF₂-O-CF(CF₃)COF (III)
in said mixture of addition products.

6. The method according to claim 5 wherein the molar amount of said monoaddition product is 90% or greater of the combined molar amount of said monoaddition product and said biaddition product in said mixture of addition products.

7. The method according to claim 5 or 6 wherein molar yield of said monoaddition product relative to moles of HFPO added is 75% or greater.

8. The method according to any fo claims 5-7 which is carried out without addition of any catalyst other than said fluoride salt.

## Patentansprüche

1. Kontinuierliches oder Repeated-Batch-Verfahren zum Herstellen einer Verbindung gemäß Formel (I)
X-R_{f}-CF₂-O-CF(CF₃)COF (I),
wobei X- für F-, FOC- oder FSO₂- steht und wobei -R_{f}- eine lineare, verzweigte oder cyclische Fluoralkengruppe mit 1 bis 20 Kohlenstoffatomen ist, die hochfluoriert ist und die Ether- und tertiäre Amingruppen beinhalten kann, umfassend die Schritte:
a) Bereitstellen eines Gemisches aus
X-R_{f}-COF (II),
wobei X- und -R_{f}- wie für Formel (I) definiert sind, einem Fluoridsalz und einem polaren Lösemittel,
b) Zugeben von Hexafluorpropylenoxid (HFPO) in einer solchen Menge, dass X-R_{f}-COF in einem mindestens 10%igen molaren Überschuss gegenüber HFPO bleibt, und Umsetzen von X-R_{f}-COF mit HFPO,
c) Abtrennen von nicht umgesetztem X-R_{f}-COF aus einem Gemisch von Additionsprodukten von Hexafluorpropylenoxid (HFPO) und X-R_{f}-COF,
d) Wiederholen von Schritt a) unter Benutzung von nicht umgesetztem X-R_{f}-COF, das in Schritt c) abgetrennt wurde.

2. Verfahren nach Anspruch 1, wobei das Gemisch von Additionsprodukten ein Monoadditionsprodukt gemäß Formel (I)
X-R_{f}-CF₂-O-CF (CF₃)COF (I)
und ein Biadditionsprodukt gemäß Formel (III)
X-R_{f}-CF₂-O-CF (CF₃) CF₂-O-CF(CF₃)COF (III)
umfasst, wobei die molare Menge an Monoadditionsprodukt in dem Gemisch von Additionsprodukten 90% oder mehr der kombinierten molaren Menge an Monoadditionsprodukt und Biadditionsprodukt beträgt.

3. Verfahren nach Anspruch 2, wobei die molare Ausbeute an dem Monoadditionsprodukt, bezogen auf die Molzahl von HFPO, die in Schritt b) zugegeben wurde, 75% oder mehr beträgt.

4. Verfahren nach einem der Ansprüche 1 bis 3, wobei Schritt b) ohne Zugabe eines von Fluoridsalz verschiedenen Katalysators durchgeführt wird.

5. Verfahren zum Umsetzen von Hexafluorpropylenoxid (HFPO) mit einem Perfluoracylfluorid gemäß der Formel
X-R_{f}-COF (II),
wobei X- für F-, FOC- oder FSO₂- steht und wobei -R_{f}- eine lineare, verzweigte oder cyclische Fluoralkengruppe mit 1 bis 20 Kohlenstoffatomen ist, die hochfluoriert ist und die Ether- und tertiäre Amingruppen enthalten kann,
zum Bilden eines Gemisches von Additionsprodukten, welches das Monoadditionsprodukt gemäß der Formel
X-R_{f}-CF₂-O-CF(CF₃)COF (I)
umfasst, wobei die molare Menge des Monoadditionsproduktes 90% oder mehr der kombinierten molaren Menge des Monoadditionsproduktes und eines Biadditionsproduktes gemäß der Formel
X-R_{f}-CF₂-O-CF (CF₃)CF₂-O-CF(CF₃)COF (III)
in dem Gemisch von Additionsprodukten beträgt.

6. Verfahren nach Anspruch 5, wobei die molare Menge des Monoadditionsproduktes 90% oder mehr der kombinierten molaren Menge des Monoadditionsproduktes und des Biadditionsproduktes in dem Gemisch von Additionsprodukten beträgt.

7. Verfahren nach Anspruch 5 oder 6, wobei die molare Ausbeute an dem Monoadditionsprodukt, bezogen auf die Molzahl von zugegebenem HFPO, 75% oder mehr beträgt.

8. Verfahren nach einem der Ansprüche 5 bis 7, das ohne Zugabe eines von Fluoridsalz verschiedenen Katalysators durchgeführt wird.

## Revendications

1. Procédé continu ou batch-répété pour la synthèse d'un composé de formule (I) :
X-R_{f}-CF₂-O-CF(CF₃)COF (I)
où X- représente F-, FOC- ou FSO₂- et où -R_{f}- est un groupement fluoroalcène linéaire, ramifié ou cyclique comprenant de 1 à 20 atomes de carbone de taux de fluoration élevé et pouvant incorporer des groupements éther ou amine tertiaire, ledit procédé comprenant les étapes suivantes :
a) se munir d'un mélange de :
X-R_{f}-COF (II)
où X- et -R_{f}- sont tels que définis pour la formule (I), un sel de type fluorure et un solvant polaire ;
b) ajouter de l'oxyde d'hexafluoropropylène (HFPO) à une teneur choisie de sorte que X-R_{f}-COF reste en excès molaire d'au moins 10 % par rapport à l'HFPO, et faire réagir X-R_{f}-COF avec l'HFPO ;
c) extraire le X-R_{f}-COF n'ayant pas réagi d'un mélange de produits d'addition de l'oxyde d'hexafluoropropylène (HFPO) et de X-R_{f}-COF ;
d) répéter l'étape a) en employant le X-R_{f}-COF n'ayant pas réagi qui a été extrait dans l'étape c).

2. Méthode selon la revendication 1 où ledit mélange de produits d'addition comprend un produit de monoaddition de formule (I) :
X-R_{f}-CF₂-O-CF(CF₃)COF (I)
et un produit de diaddition de formule (III) :
X-R_{f}-CF₂-O-CF(CF₃)CF₂-O-CF(CF₃)COF (III)
où la teneur en moles de produit de monoaddition dans ledit mélange de produits d'addition est supérieure ou égale à 90 % de la somme des quantités molaires dudit produit de monoaddition et dudit produit de diaddition.

3. Méthode selon la revendication 2 où le rendement molaire dudit produit de monoaddition par rapport au nombre de moles d'HFPO ajoutées dans l'étape b) est supérieur ou égal à 75 %.

4. Méthode selon l'une quelconque des revendications 1 à 3 où l'étape b) s'effectue sans ajout de quelque catalyseur que ce soit autre que ledit sel de type fluorure.

5. Méthode de réaction de l'oxyde d'hexafluoropropylène (HFPO) avec un fluorure de perfluoroacyle de formule :
X-R_{f}-COF (II)
où X- représente F-, FOC- ou FSO₂- et où -R_{f}- est un groupement fluoroalcène linéaire, ramifié ou cyclique comprenant de 1 à 20 atomes de carbone de taux de fluoration élevé et pouvant incorporer des groupements éther ou amine tertiaire,
dans le but de former un mélange de produits d'addition comprenant le produit de monoaddition de formule :
X-R_{f}-CF₂-O-CF(CF₃)COF (I)
la teneur en moles dudit produit de monoaddition étant supérieure ou égale à 90 % de la somme des quantités molaires dudit produit de monoaddition et d'un produit de diaddition de formule :
X-R_{f}-CF₂-O-CF (CF₃) CF₂-O-CF(CF₃) COF (III)
dans ledit mélange de produits d'addition.

6. Méthode selon la revendication 5 où la teneur en moles de produit de monoaddition est supérieure ou égale à 90 % de la somme des quantités molaires dudit produit de monoaddition et dudit produit de diaddition dans ledit mélange de produits d'addition.

7. Méthode selon la revendication 5 ou 6 où le rendement molaire dudit produit de monoaddition par rapport au nombre de moles d'HFPO ajoutées est supérieur ou égal à 75 %.

8. Méthode selon l'une quelconque des revendications 5 à 7 menée sans ajout de quelque catalyseur que ce soit autre que ledit sel de type fluorure.
